# EUROPEAN PATENT APPLICATION

(11) **EP 3 184 103 A1**
(43) Date of publication of application: **28.06.2017**
(21) Application number: 15201694.5
(22) Date of filing: 21.12.2015
(51) Int. Cl.: A61K 9/20, A61K 31/40

(54) **PHARMACEUTICAL COMPOSITION COMPRISING ATORVASTATIN OR A SALT THEREOF**

(71) Applicant: Hexal AG, 83607 Holzkirchen (DE)
(72) Inventor: STRIEGL, Barbara, 83607 Holzkirchen (DE); OTTO, Ina, 83607 Holzkirchen (DE); KOPAL, Alexandra, 83607 Holzkirchen (DE)
(74) Representative: Prüfer & Partner mbB Patentanwälte · Rechtsanwälte

(57) **Abstract**

The present invention relates to a solid pharmaceutical composition comprising atorvastatin or a salt thereof, in particular to a composition having the total weight of less than or equal to 600 mg and a tablet comprising said composition. The present invention furthermore relates to a process for the preparation of solid composition comprising atorvastatin or a salt thereof and its use as a medicament, especially for the treatment of hypercholesterolemia or hyperlipidemia.

## Description

### Field of Invention

The present invention relates to a solid pharmaceutical composition comprising atorvastatin or a salt thereof, in particular to a solid composition with stabilized atorvastatin component and having a purposively limited total weight, and a tablet comprising said composition. The present invention furthermore relates to a process for the preparation of solid composition comprising atorvastatin or a salt thereof and its use as a medicament, especially for the treatment of hypercholesterolemia or hyperlipidemia.

### Description of Background Art

Atorvastatin, (3R,5R)-7-[2-(4-Fluorophenyl)-3-phenyl-4-(phenylcarbamoyl)-5-propan-2-ylpyrrol-1-yl]-3,5-dihydroxyheptanoic acid, was first described in EP 0330057. Atorvastatin is a member of the class of drugs called statins which are therapeutically effective drugs for the treatment of hyperlipidemia and hypercholesterolemia, both of which are risk factors for arteriosclerosis and coronary heart diseases. Atorvastatin acts by suppressing cholesterol biosynthesis by competitively inhibiting 3-hydroxy-3-methyl-glutaryl-coenzyme A reductase.

It is known from the prior art that atorvastatin has an insufficient stability, i.e. it is susceptible to heat, humidity, low pH and light, and UV radiation. Hence, various different approaches have been made to stabilize atorvastatin in a composition. EP 680320 described of using alkaline earth metal salt stabilizers, such as calcium, magnesium, and lithium salts of hydroxides, oxides or carbonates. Said alkali metal salt additives used for stabilizing atorvastatin in the composition according to EP 680320 are virtually insoluble and therefore known to be difficult to be processed in order to give a composition wherein they evenly surround the active ingredient. Hence, a solution of using a smaller amount of alkali metal salt stabilizer was described in WO 03/068191.

WO 04/032920 and WO 05/011638, on the other hand, described another solution of stabilizing atorvastatin by exposing it to an inert gas atmosphere having low oxygen partial pressure. EP 1986608 furthermore disclosed compositions of atorvastatin that were stabilized by using sodium hydroxide as alkali metal additive which were exposed to an atmosphere comprising 1 to 16 % by volume of oxygen.

Other different attempts, such as using buffering agents capable of providing a pH in the range from 7 to 11 were also described in WO 00/35425. US 2012/0165386 also described a stable atorvastatin composition comprising an alkalizing agent having the buffering capacity of between 2 to 5.

The US marketed product, Lipitor, exists in four different strengths, namely 10 mg, 20 mg, 40 mg and 80 mg strength. The high dose strength comprising 80 mg of atorvastatin has a total tablet weight of 1200 mg and is 10.4 mm wide and 19.3 mm long. The dimension of this tablet is thus relatively big and may thus cause problems for the patients when swallowing the tablet.

Although many different solutions of a composition comprising atorvastatin were suggested by the prior art, there is still an unmet need of providing a stable composition of atorvastatin in a simple and economical manner which enables easier swallowing for the patient.

Therefore, it is the object of the present invention to provide a stable solid composition of atorvastatin or a pharmaceutically acceptable salt thereof which is economical and in the same time more "patient friendly".

### Detailed Description of the Invention

The present invention provides a solid pharmaceutical composition of atorvastatin or a pharmaceutically acceptable salt having the total weight of the composition of less than or equal to 600 mg, and the process of preparing thereof, the tablets comprising said composition and the use of the composition and the tablets for the treatment of hypercholesterolemia or hyperlipidemia.

In particular, the present invention provides a solid pharmaceutical composition comprising from 10 wt % to 20 wt % of atorvastatin or a pharmaceutically acceptable salt thereof, from 5 wt % to 60 wt % of an additive selected from group consisting of alkali metal salts and alkaline earth metal salts, and at least one further pharmaceutically acceptable excipient, wherein the total weight of the composition is less than or equal to 600 mg.

It was surprisingly found that a solid composition, when comprising from 10 wt % to 20 wt % atorvastatin or a pharmaceutically acceptable salt thereof, from 5 wt % to 60 wt % of an additive, and a further excipient, can be formulated in a robust manner even when having only a limited total weight of the composition of less than or equal to 600 mg. Even more surprisingly, the composition of the present invention, with its controlled ingredient amounts and and with its limited total amount despite of the need for a sufficient amount of the stabilizing additive, allowed for forming tablet cores, which correspondingly are smaller in weight and hence, are easier to swallow and more economical, while in the same time fulfill all relevant requirements, such as content and mass uniformity, disintegration, friability and abrasion. Thus, it was unexpectedly found that a limited total weight of the composition of less than or equal to 600 mg or even less total weight can be achieved without a detriment to atorvastatin stabilization while maintaining or even improving relevant use attributes of appropriately prepared dosage forms, notably in the form of tablets.

Preferably, the present invention provides a solid composition having the total weight of from 300 mg to 600 mg, preferably from 450 mg to 550 mg, most preferably is about 500 mg.

The term "composition" and the "total weight" thereof (corresponding to 100 wt %), if not specified otherwise, means a pharmaceutical composition component or unit which contains the atorvastatin as the active ingredient, such as cores, pellets or granules. Further components or compartments may optionally be applied to such basic composition, and then its weight is added to the indicated total weight of the basic composition, for example a coating coated onto the atorvastatin containing composition component or unit, or another layer or unit not containing atorvastatin.

Suitable pharmaceutically acceptable salts of atorvastatin include salts of inorganic and organic acids. Preferably, the pharmaceutically acceptable salt of atorvastatin is selected from the group consisting of calcium, magnesium, potassium, aluminium, iron and zinc. Most preferably, atorvastatin salt is atorvastatin calcium.

Atorvastatin or a pharmaceutically acceptable salt thereof may be present in the composition of the present invention in a solid crystalline or amorphous form. Most preferably, atorvastatin calcium is selected and is in the polymorphic form I described in WO 97/03959 or in an amorphous form.

Preferably, said atorvastatin or a pharmaceutically acceptable salt thereof having particle size distribution d(90) of less than 150 µm, preferably less than 100 µm, more preferably less than 50 µm, most preferably less than 25 µm.

The term d(90) as used according to the present invention relates to the maximum particle diameter of the atorvastatin or a pharmaceutically acceptable salt thereof below which 90 % of a sample volume comprising the atorvastatin or a pharmaceutically acceptable salt thereof exists.

The amount of atorvastatin or a pharmaceutically acceptable salt thereof in the composition of the present invention is 10 wt % to 20 wt %, the preferred amount is from 12 wt % to 18 wt %, most preferably about 15 wt %. Micronized atorvastatin or a pharmaceutically acceptable salt thereof in said high weight ratio in the composition is known to show very poor flow properties. Hence, it was surprisingly found that a composition comprising 10 wt % to 20 wt % of atorvastatin or a pharmaceutically acceptable salt thereof and having the limited total weight of the composition of less than or equal to 600 mg could be prepared, that allow for forming the tablet cores which exhibit the required content and mass uniformity, disintegration, friability and abrasion.

The pharmaceutical composition of the present invention may include one or more of a 10 mg, 20 mg, 40 mg or 80 mg dosage unit (noting that the dosage strengths are approximated amounts of about the respective value) of atorvastatin or a pharmaceutically acceptable salt thereof. In case of using a pharmaceutically acceptable salt, the amount of the atorvastatin compound itself shall be calculated and meant by the present mass indications, i.e. calculating without the salt former. Preferably, the composition of the present invention comprises of about 80 mg of atorvastatin or a pharmaceutically acceptable salt thereof and has a total weight of the composition of from 300 mg to 600 mg, more preferably of from 450 to 550 mg, most preferably of about 500 mg. In the most preferred embodiment the present invention relates to the composition having an amount of atorvastatin or a pharmaceutically acceptable salt thereof of about 80 mg and has the total weight of composition of about 500 mg.
In another preferred embodiment the solid composition of the present invention comprises of about 40 mg of atorvastatin or a pharmaceutically acceptable salt thereof and has a total weight of composition of less than or equal to 300 mg, preferably from 150 mg to 300 mg, more preferably from 200 to 275 mg, most preferably about 250 mg. In another preferred embodiment of the present invention the solid composition comprises of about 20 mg amount of atorvastatin or a pharmaceutically acceptable salt thereof and has a total weight of the composition of less than or equal to 150 mg, preferably from 100 mg to 150 mg, more preferably from 110 to 140 mg, most preferably about 125 mg. In yet another preferred embodiment of the present invention the solid composition comprises of about 10 mg of atorvastatin or a pharmaceutically acceptable salt thereof and has a total weight of the composition of less than or equal to 75 mg, preferably from 50 mg to 100 mg, more preferably from 50 to 75 mg, most preferably about 62.5 mg.

The term "about" generally means within 10%, preferably 5% and more preferably within 1% of a given value or range. Alternatively, the term "about" means within an acceptable standard error of the mean, when considered by one of the ordinary skill in the art. Apart from using the term "about", preferably the amount indications are accurately met.

The solid pharmaceutical composition of the present invention further includes an additive, selected from group consisting of alkali metal salts and alkaline earth metal salts, which is needed to stabilize atorvastatin or a pharmaceutically acceptable salt thereof in the composition. According to one aspect of the present invention the composition comprises the additive selected from the group consisting of carbonates, bicarbonates, hydroxides, silicates, phosphates of sodium or calcium, and combinations thereof. Preferably, the additive is selected from the group consisting of sodium carbonate, sodium bicarbonate, sodium hydroxide, sodium silicate, disodium hydrogen orthophosphate, sodium dihydrogen phosphate, disodium hydrogen phosphate, sodium phosphate, sodium aluminate, calcium carbonate, calcium bicarbonate, calcium hydroxide, calcium silicate, calcium hydrogen orthophosphate, calcium hydrogen phosphate, dicalcium phosphate, calcium phosphate, calcium aluminate, or combinations thereof. More preferably the additive is selected from the group consisting of carbonates or bicarbonates of sodium or calcium, and combinations thereof; most preferably the additive is sodium carbonate or calcium carbonate.

The amount of the additive in the composition of the present invention is from 5 wt % to 60 wt %, preferably from 5 wt % to 30 wt %. More preferably, the amount of the additive in the composition from 8 wt % to 30 wt %, more preferably 8 wt % to 25 wt %, most preferably is about 10 wt %.

It is known form the art that additives, such as carbonates or bicarbonates of sodium or calcium, in particular when in high amounts, are virtually insoluble and therefore difficult to be processed to give a composition wherein they evenly surround the active ingredient. Thus, it was surprisingly found that the compositions of the present invention comprising from 5 wt % to 60 wt % of the additive and having the limited total weight of the composition of less than or equal to 600 mg could be prepared. It was even more surprising that said compositions allowed for forming the tablet cores which exhibit the required content and mass uniformity, disintegration, friability and abrasion.

According to a preferred embodiment of the present invention the composition comprises at least one further pharmaceutically acceptable excipient selected from the group consisting of a filler, a disintegrant, a binder, a glidant, a lubricant, and a surfactant, and optionally further an antioxidant, a sweetener, a chelating agent; and combinations thereof. An excipient functioning as both a filler and a binder is also possible and may be preferred.

The amount of the at least one further excipient in the composition of the present invention may be from 20 wt % to 85 wt %. Preferably, the amount of the at least one further excipient is from 50 wt % to 85 wt %, more preferably from to 60 wt % to 80 wt %, most preferably about 75 wt %.

In one particularly preferred embodiment of the present invention, the at least one further pharmaceutically acceptable excipient is a filler, wherein the filler may additionally function as a binder. The fillers may be present in the form of a single compound or in the form of a mixture of compounds. Suitable pharmaceutically acceptable fillers include lactose, granulated forms of lactose such as Tablettose^{™}, microcrystalline cellulose, silicified microcrystalline cellulose, powdered cellulose, lactose monohydrate, calcium carbonate, dibasic calcium phosphate, tribasic calcium phosphate, sucrose, glucose, dextrates, dextrins, dextrose, fructose, lactitol, mannitol, sorbitol, compressible sugar, starch, pregelatinized starch, and/or mixtures of the foregoing. The most preferred filler is microcrystalline cellulose and lactose as well as hydrates and granulated forms of lactose, and/or the combination thereof. Especially if microcrystalline cellulose is chosen, additionally a binder function can preferably be accomplished.

The amount of filler, as a single or multiple component, if contained in the pharmaceutical composition can constitute up to 80 wt %, preferably from 20 wt % to 70 wt %, more preferably from 30 wt % to 70 wt % in total weight of composition.

According to a further embodiment of the present invention, the at least one further pharmaceutically acceptable excipient is a disintegrant. Suitable disintegrants are selected from croscarmellose sodium, crospovidone, sodium starch glycolate, corn starch, potato starch, maize starch and modified starches, calcium silicates, low substituted hydroxypropylcellulose and the like, and combinations thereof. Preferably, the disintegrant is sodium starch glycolate or croscarmellose sodium.

Preferably the disintegrant is used together with the above described filler as combined excipients, in order to balance processability and formulation performances with dissolution properties of the pharmaceutical composition of the present invention.

The amount of the disintegrant, if contained in the pharmaceutical composition, can be up to 15 wt%, preferably constitutes from 2 wt % to 10 wt %, more preferably from 2 wt % to 8 wt % in total weight of the composition.

In yet a further embodiment of the present invention the at least one further pharmaceutically acceptable excipient according to the present invention may be a binder. Suitable binders are selected from hydroxypropyl cellulose (HPC), hypromellose (hydroxypropyl methylcellulose, HPMC), microcrystalline cellulose, acacia, alginic acid, carboxymethylcellulose, ethylcellulose, methylcellulose, hydroxaethylcellulose, ethylhydroxyethylcellulose, polyvinyl alcohol, polyacrylates, carboxymethylcellulose calcium, carboxymethylcellulose sodium, compressible sugar, ethylcellulose, gelatin, liquid glucose, methylcellulose, polyvinylpyrrolidone (PVP) and pregelatinized starch, the most preferred being HPC and PVP.

Preferably the binder is used together with the above described filler and/or disintegrant as combined excipients, in order to even better balance processability and formulation performances with dissolution properties of the pharmaceutical composition of the present invention.

The amount of the binder, if contained in the pharmaceutical composition, can be up to 15 wt%, preferably constitutes from 0.5 wt % to 8 wt %, more preferably 0.5 wt % to 6 wt % in the total weight of the composition.

According to a further embodiment of the present invention, the at least one pharmaceutically acceptable excipient may be a lubricant. Lubricants are selected from the group consisting of magnesium stearate, magnesium lauryl sulphate and sodium stearyl fumarate, sucrose esters of fatty acids, polyethylene glycol and stearic acid. Preferably, the lubricant is magnesium stearate.
Preferably the lubricant is used together with the above described filler, disintegrant and/or binder as combined excipients.

The amount of the lubricant, if contained in the pharmaceutical composition, can be up to 3 wt %, preferably constitutes from 0.2 wt % to 2.5 wt % in the total weight of the composition.

The at least one pharmaceutically acceptable excipient may be a glidant. Glidants are preferably selected from the group consisting of silicon dioxide, talc and aluminium silicate. Most preferred glidants are fumed silica and Syloid FP silica.

Preferably the glidant is used together with the above described filler. More preferably the glidant is used together with the above described filler, disintegrant, binder and/or lubricant as combined excipients.

The amount of the glidant contained in the pharmaceutical composition can be up to 3 wt %, preferably from 0.5 wt % to 2 wt % in the total weight of the composition.

In a particularly preferred embodiment, the pharmaceutical composition is a compressed atorvastatin containing unit such as a compressed tablet core, and contains besides the specified stabilizing additive at least the further excipients filler, binder and disintegrant, and further preferably additionally contains lubricant and glidant.

According to a further embodiment of the present invention, the at least one pharmaceutically acceptable excipient may be a surfactant. Suitable surfactants include emulsifying agents such as those commonly known to one skilled in the art. Suitable surfactants include but are not limited to heptadecaethylene oxycetanol, lecithins, sorbitol monooleate, polyoxyethylene sorbitol monooleate, polyoxyethylene stearate, polyoxyethylen sorbitan monolaurate, benzalkonium chloride, nonoxynol 10, oxtoxynol 9, polysorbates for example 20, 40, 60 or 80, sorbitan monoalmitate, sodium salts of fatty alcohol-sulaftes such as sodium lauryl sulfate, sodium dodecylsulfate, sodium salts of sulfosuccinates, such as sodium dioctylsulfosuccinate, partially esters of fatty acids with alcohols such as glycerine monostearate, partially esters of fatty acids with sorbitans such as sorbitan monolaurate, partially esters of fatty acids with polyhydroxyethylene sorbitans such as polyethyleneglycol sorbitan monolaurate,-monostearate or -monooleate, ethers of fatty alcohols with polyhydroxyethylene, esters of fatty acids with polyhydroxyethylene, copolymers of ethylenoxide and propylenoxide (Piuronic^{®}) and ethoxylated triglycerides. The most preferred are polyoxyethylene sorbitan fatty acid esters.

The amount of the surfactant, if contained in the pharmaceutical composition, can be up to 15 wt %, preferably from 0.1 wt % to 10 wt %, most preferably from 1 wt % to 5 wt % in the total weight of the composition.

Suitable and further optional pharmaceutically acceptable excipients are also an antioxidant, a sweetener, a chelating agent, a flavouring agent, a colouring agent, or an opacifying agent and a pigment. Suitable antioxidants include, but are not limited to, vitamin E acetate,-tocopherol, ascorbyl palmitate, butylated hydroxyanisole (BHA), butylated hydroxytoluene (BHT), propyl gallate, dithiotreitol, or tocopherol polyethyleneglycol succinate (TPGS). Chelating agents can also be used as antioxidants, for example EDTA or cyclodextrins.

In a preferred embodiment of the present invention the at least one further pharmaceutically acceptable excipient is a filler having mean particle size of more than 50 µm, preferably more than 70 µm and more preferably more or equal to 100 µm. Preferably, said filler is microcrystalline cellulose, lactose, or combinations thereof, having mean particle size of more than 50 µm, preferably more than 70 µm and more preferably more or equal to 100 µm. In the most preferred embodiment the at least one further pharmaceutically acceptable excipient is a filler having mean particle size of more than 50 µm, preferably more than 70 µm and more preferably more or equal to 100 µm, and a glidant having a high bulk density, such as Syloid FP silica.

It has been surprisingly found that correspondingly chosen types of the filler which meet the preferred size criteria have a favourable effect on the processability and formulation performance of the pharmaceutical composition for allowing the desired limitation of the total weight. Accordingly, selecting relatively coarser types of respective filler substances, which meet the specified preferred mean particle size criteria, is particularly beneficial compared to using smaller and finer size types which do not meet the specified size criteria.

In a further preferred embodiment, the composition of the present invention may have a particle size distribution d(50) equal or higher than 75 µm, preferably 100 µm, more preferably 110 µm.

The term "particle size distribution d(50)" as used according to the present invention relates to the maximum particle diameter of the composition below which 50 % of a sample volume comprising the composition exists. The method by which the d(50) is determined according to the present invention is described in detail in under Examples below.

Due to the physical properties of the additives in the composition, such as carbonates or bicarbonates of sodium or calcium, a problem of inferior flow properties of the compression mass may occur during the process of forming the tablet cores. This problem is in particular pronounced in smaller dosage strengths, such as the 10 mg strength, which typically shall have a composition that is proportional to higher dosage strengths, such as the 80 mg strength, therefore, comprising from 5 wt % to 60 wt % of an additive. It was surprisingly found that when the composition of the present invention comprises a filler having a mean particle size of more than 50 µm, preferably more than 70 µm and more preferably more or equal to 100 µm the flow properties of the compression mass were markedly improved. The flow properties of the compression mass were improved even further when adding a glidant having larger particle size and higher bulk density, such as Syloid FP silica to the compression mass comprising the filler having a mean particle size of more than 50 µm, preferably more than 70 µm and more preferably more or equal to 100 µm. Even more surprisingly it was found that the compositions of the present invention having a particle size distribution d(50) equal or higher than 75 µm, preferably 100 µm, more preferably 110 µm exhibit improved flow properties of the compression mass.

In one aspect, the composition of the present invention may be in the form of compressed tablet cores or in the form of pellets, or in the form of granules.

In another aspect, the present invention provides a process for preparing cores, pellets or granules comprising the composition of the present invention. The process for preparing the cores, pellets or granules comprising composition of the present invention comprises the steps of
a) providing atorvastatin or a pharmaceutically acceptable salt thereof, an additive selected from group consisting of alkali metal salts and alkaline earth metal salts, and at least one further pharmaceutically acceptable excipient;
b) mixing the components of step a);
c) preparing cores, pellets or granules comprising the composition.

With respect to step a) of said process, reference is made to the above description on atorvastatin or a pharmaceutically acceptable salt thereof, on the stabilizing additive, and on the at least one further pharmaceutically acceptable excipient which respectively apply here as well.

In a preferred embodiment, step c) of said process involves direct compression for preparing cores, pelletization for preparing pellets, or granulation for preparing granules.

The term "direct compression" used herein means a preparation wherein dry composition containing the active principle atorvastatin or a pharmaceutically acceptable salt thereof and the above specified further ingredients for a pharmaceutical dosage form are processed by applying of a sufficient force by punches of a tablet press on a powder to compact it into a tablet core. In a preferred embodiment the direct compression process of the present invention includes the steps of mixing and sieving of atorvastatin or a pharmaceutically acceptable salt thereof with a filler, a stabilizing additive, a glidant, a binder and a disintegrant; blending the mass; lubricating the blend with a lubricant; and compressing the blend to form tablet cores.
In one preferred embodiment, the granulation method used for preparing the cores of step c) is selected from the group consisting of dry granulation or wet granulation.

The term "dry granulation" used herein means a preparation wherein the dry formulation contains the active principle atorvastatin or a pharmaceutically acceptable salt thereof and further ingredients for a pharmaceutical dosage form have been processed by compacting into compacts with roller compactor or tabletting machine by using slugging tooling or regular tableting tooling and subsequently milling - crushing and sizing these compacts into dry granulate. The term "dry granulation" may also include moisture-activated dry granulation as further described below.

The process of wet granulation is known to the person skilled in the art and includes wet granulation using a high shear granulator and wet granulation using a fluid bed granulator, i.e. fluid bed granulation. In a preferred embodiment the granulation process of the present invention is a fluid bed granulation. In a particularly preferred embodiment the wet granulation process of the present invention included the steps of preparing the granulation solution of a binder dissolved in water; spraying said solution on the blend comprising atorvastatin or a pharmaceutically acceptable salt thereof, a filler, a stabilizing additive, and a disintegrant; sieving the granules; lubricating the compression mass with a lubricant to form a blend; and compressing the blend to form tablet cores. The process of the present invention can be carried out in a fluidized bed granulator.

The term "pelletization" used herein means a process of agglomeration (size-enlargement) that converts fine powders or particles of bulk drugs and excipients into small, free-flowing, more or less spherical units, i.e. pellets. Pelletization is referred to as a size-enlargement process that involves the manufacture of agglomerates, i.e. pellets, with a relatively narrow size range, usually with a mean size from 0.5 to 2.0 mm. Said pellets have free-flowing properties and a low porosity.

The pharmaceutical composition according to the present invention is in a solid form, including tablets, capsules, caplets, lozenges and sachets. Tablets may be suitably coated. Capsule formulations may cover both soft and hard capsules.

A pharmaceutical composition according to the present invention is preferably embodied in an oral dosage form, more preferably is in the form of a tablet, particularly where the composition forms a tablet core which further comprises a coating.

The term "coating" as used herein refers to a layer which completely covers an object and is applied by film coating. Film-forming agents and coating materials include, but are not limited to liquid glucose, hydroxyethyl cellulose, hydroxypropyl cellulose (HPC), hydroxypropyl methylcellulose (hypromellose, HPMC), methylcellulose, ethylcellulose, cellulose acetate phthalate, shellac, polyvinylpyrrolidone, copolymers of vinylpyrrolidone and vinyl acetate such as Kollidon^{®} VA64 BASF, copolymers of acrylic- and/or methacrylic acid esters with trimethylammoniummethylacrylate, copolymers of dimethylaminomethacrylic acid and neutral methacrylic acid esters, polymers of methacrylic acid or methacrylic acid esters, copolymers of acrylic acid ethylester and methacrylic acid methyl ester, and copolymers of acrylic acid and acrylic acid methylester. The preferred film forming polymers are HPMC. The coating can be selected from the group of ready to form dispersion such as OPADRY. The coating dispersion may further include plasticizers, such as polyethylene glycol (PEG), diethyl phthalate and glycerol, preferably the plasticizer is PEG. Further excipients may be added to the coating dispersion, such as titanium dioxide, colorants and may optionally include other excipients such as anti-tacking agents. Any method for film coating, known in the field of the pharmaceutical technology, may be used.

In the preferred embodiment, the tablet of the present invention has a coating and in this form has a diameter of less than or equal to 12.0 mm.

The dimensions of a tablet are dependent on the amount of the active agent needed for an effective therapy and the amounts of the excipients. Type and amount of the excipients, in combination with the process for preparing, influence the properties such as friability and the abrasion of the tablet cores, disintegration, content and mass uniformity of the tablets, and for bioavailability of the compound in mammals. Owing to the limitation of total weight and the achievement of the aforementioned tablet properties, it became possible that the tablets of the present invention can have a small size of the diameter of less than or equal to 12.0 mm. This is in particular surprising, as the tablets of the present invention comprise additives, such as carbonates or bicarbonates of sodium or calcium and a high weight amount of atorvastatin or a pharmaceutically acceptable salt thereof, which are due to their physical properties difficult to be processed. Even more surprisingly, the tablets of the present invention still exhibit good tablet properties, such as content uniformity, mass uniformity and disintegration.

According to another aspect the composition or the tablet of the present invention may be used in the treatment of hypercholesterolemia or hyperlipidemia.

### Examples

Examples 1 to 8 were prepared using direct compression method.

In each of Examples 1 to 8, atorvastatin calcium was mixed and sieved together with microcrystalline cellulose, calcium carbonate, silicon dioxide, polyvinylpyrrolidone and sodium starch glycolate with a total blending time of 25 min. Then the compression mass was lubricated with sieved magnesium stearate for another 5 min and the resulting blend was compressed into tablet cores with a standard rotary compression machine. All components for film coating were dissolved in water as solvent and then sprayed on the obtained tablet cores.

### Example 1:

| | **Function** | **Amount (mg)** |
|---|---|---|
| **Cores** | | |
| Atorvastatin Calcium | Active ingredient | 82.72* |
| Microcrystalline cellulose (Avicel PH 102)** | Filler | 307.28 |
| Calcium carbonate | Additive | 50.00 |
| Silicon dioxide (Aerosil 200) | Glidant | 7.60 |
| Polyvinylpyrrolidone (Kollidon 25) | Binder | 6.80 |
| Sodium Starch Glycolate (Primojel) | Disintegrant | 33.60 |
| Magnesium Stearate | Lubricant | 12.00 |
| | | **500.00** |
| **Film/Coating** | | |
| Hydroxypropylmethylcellulose (Pharmacoat 606) | Coating polymer | 3.40 |
| Polyethylene glycol (PEG 6000 Powder) | Plasticizer | 1.00 |
| Titanium Dioxide | Coloring agent | 0.50 |
| Tween 80 | Surfactant | 5.10 |
| Water | Solvent | q.s. |
| | | **10.00** |
| **Sum** | | **510.00** |

| | | |
|---|---|---|
| * corresponding to 80 mg of atorvastatin substance without calculating calcium ** mean particle size of 100 µm | | |

The cores of the Example 1 had the friability of 0.19 % and an abrasion of 0.37 %. The PSD d(50) of the compression mass was 108 µm.

The tablets of Example 1 exhibit the mass variation of 99.4 %, content uniformity of 100.8 %, and a disintegration time of less than 2 min. The average weight of 20 tablets was 506.2 mg. Uniformity of mass conformed with the acceptance criteria defined in Ph. Eur.2.9.40. The diameter of the tablet was 11.6 mm, and the height of the tablet was 5.2 mm.

### Example 2:

| | **Function** | **Amount (mg)** |
|---|---|---|
| **Core** | | |
| Atorvastatin Calcium | Active ingredient | 82.72* |
| Microcrystalline cellulose (Avicel PH 102)** | Filler | 335.68 |
| Sodium carbonate | Additive | 40.0 |
| Silicon dioxide (Aerosil 200) | Glidant | 7.60 |
| Polyvinylpyrrolidone (Kollidon 25) | Binder | 7.00 |
| Sodium Starch Glycolate (Primojel) | Disintegrant | 15.00 |
| Magnesium Stearate | Lubricant | 12.00 |
| | | **500.00** |
| **Film/Coating** | | |
| Hydroxypropylmethylcellulose (Pharmacoat 606) | Coating polymer | 7.00 |
| Polyethylene glycol (PEG 6000 Powder) | Plasticizer | 2.00 |
| Titanium Dioxide | Coloring agent | 1.00 |
| Water | Solvent | q.s. |
| | | **10.00** |
| **Sum** | | **510.00** |

| | | |
|---|---|---|
| * corresponding to 80 mg of atorvastatin substance without calculating calcium ** mean particle size of 100 µm | | |

The cores of the Example 2 had the friability of 0.11 % and an abrasion of 0.26 %. The PSD d(50) of the compression mass was 107 µm.

The tablets of Example 2 exhibit the mass variation of 99.7 %, content uniformity of 100.9 %, and a disintegration time of less than 3 min. The average weight of 20 tablets was 499.4 mg. Uniformity of mass conformed with the acceptance criteria defined in Ph. Eur.2.9.40. The diameter of the tablet was 11.6 mm, and the height of the tablet was 5.4 mm.

### Example 3:

| | **Function** | **Amount (mg)** |
|---|---|---|
| **Core** | | |
| Atorvastatin Calcium | Active ingredient | 82.72* |
| Microcrystalline cellulose (Emcocel 90)** | Filler | 320.18 |
| Calcium carbonate | Additive | 50.00 |
| Silicon dioxide (Aerosil 200) | Glidant | 7.60 |
| Polyvinylpyrrolidone (Kollidon 25) | Binder | 12.50 |
| Sodium Starch Glycolate (Primojel) | Disintegrant | 15.00 |
| Magnesium Stearate | Lubricant | 12.00 |
| | | **500.00** |
| **Film/Coating** | | |
| Hydroxypropylmethylcellulose (Pharmacoat 606) | Coating polymer | 7.00 |
| Polyethylene glycol (PEG 6000 Powder) | Plasticizer | 2.00 |
| Titanium Dioxide | Coloring agent | 1.00 |
| Water | Solvent | q.s. |
| | | **10.00** |
| **Sum** | | **510.00** |

| | | |
|---|---|---|
| * corresponding to 80 mg of atorvastatin substance without calculating calcium ** mean particle size of 133 µm | | |

The cores of the Example 3 had the friability of 0.02 % and an abrasion of 0.01%.

The tablets of Example 3 exhibit the mass variation of 97.3 %, content uniformity of 97.3%, and a disintegration time of 4.3 min. The average weight of 20 tablets was 506.3 mg. Uniformity of mass conformed with the acceptance criteria defined in Ph. Eur.2.9.40. The diameter of the tablet was 11.6 mm, and the height of the tablet was 4.9 mm.

### Example 4:

| | **Function** | **Amount (mg)** |
|---|---|---|
| **Core** | | |
| Atorvastatin Calcium | Active ingredient | 82.72* |
| Microcrystalline cellulose (Emcocel 90)** | Filler | 317.68 |
| Calcium carbonate | Additive | 50.00 |
| Silicon dioxide (Aerosil 200) | Glidant | 7.60 |
| Polyvinylpyrrolidone (Kollidon 25) | Binder | 20.00 |
| Sodium Starch Glycolate (Primojel) | Disintegrant | 10.00 |
| Magnesium Stearate | Lubricant | 12.00 |
| | | **500.00** |
| **Film/Coating** | | |
| Hydroxypropylmethylcellulose (Pharmacoat 606) | Coating polymer | 7.00 |
| Polyethylene glycol (PEG 6000 Powder) | Plasticizer | 2.00 |
| Titanium Dioxide | Coloring agent | 1.00 |
| Water | Solvent | q.s. |
| | | **10.00** |
| **Sum** | | **510.00** |

| | | |
|---|---|---|
| * corresponding to 80 mg of atorvastatin substance without calculating calcium ** mean particle size of 133 µm | | |

The cores of the Example 4 had the friability of 0.04 % and an abrasion of 0.07 %.

The tablets of Example 4 exhibit the mass variation of 97.8 %, content uniformity of 97.8%, and a disintegration time of 6.1 min. The average weight of 20 tablets was 510.2 mg. Uniformity of mass conformed with the acceptance criteria defined in Ph. Eur.2.9.40. The diameter of the tablet was 11.6 mm, and the height of the tablet was 5.1 mm.

### Example 5:

| | **Function** | **Amount (mg)** |
|---|---|---|
| **Core** | | |
| Atorvastatin Calcium | Active ingredient | 82.72* |
| Microcrystalline cellulose (Avicel PH 102)** | Filler | 317.68 |
| Calcium carbonate | Additive | 50.00 |
| Silicon dioxide (Aerosil 200) | Glidant | 7.60 |
| Polyvinylpyrrolidone (Kollidon 25) | Binder | 20.00 |
| Sodium Starch Glycolate (Primojel) | Disintegrant | 10.00 |
| Magnesium Stearate | Lubricant | 12.00 |
| | | **500.00** |
| **Film/Coating** | | |
| Hydroxypropylmethylcellulose (Pharmacoat 606) | Coating polymer | 7.00 |
| Polyethylene glycol (PEG 6000 Powder) | Plasticizer | 2.00 |
| Titanium Dioxide | Coloring agent | 1.00 |
| Water | Solvent | q.s. |
| | | **10.00** |
| **Sum** | | **510.00** |

| | | |
|---|---|---|
| * corresponding to 80 mg of atorvastatin substance without calculating calcium ** mean particle size of 100 µm | | |

The cores of the Example 5 had the friability of 0.05 % and an abrasion of 0.08 %. The PSD d(50) of the compression mass was 83 µm.

### Example 6:

| | **Function** | **Amount (mg)** |
|---|---|---|
| **Core** | | |
| Atorvastatin Calcium | Active ingredient | 82.72* |
| Microcrystalline cellulose (Avicel PH 200)** | Filler | 317.68 |
| Calcium carbonate | Additive | 50.00 |
| Silicon dioxide (Syloid 244) | Glidant | 7.60 |
| Polyvinylpyrrolidone (Kollidon 25) | Binder | 20.00 |
| Sodium Starch Glycolate (Primojel) | Disintegrant | 10.00 |
| Magnesium Stearate | Lubricant | 12.00 |
| | | **500.00** |
| **Film/Coating** | | |
| Hydroxypropylmethylcellulose (Pharmacoat 606) | Coating polymer | 7.00 |
| Polyethylene glycol (PEG 6000 Powder) | Plasticizer | 2.00 |
| Titanium Dioxide | Coloring agent | 1.00 |
| Water | Solvent | q.s. |
| | | **10**.**00** |
| **Sum** | | **510.00** |

| | | |
|---|---|---|
| * corresponding to 80 mg of atorvastatin substance without calculating calcium ** mean particle size of 180 µm | | |

The cores of the Example 6 had the friability of 0.15 % and an abrasion of 0.33 %. The PSD d(50) of the compression mass was 191 µm.

The average weight of 20 tablets was 510.3 mg. Uniformity of mass conformed with the acceptance criteria defined in Ph. Eur.2.9.40.

Examples 7 to 10 were prepared using fluid bed granulation method. In each of Examples 7 to 10, a granulation solution consisting of hydroxypropylcellulose and Tween 80 dissolved in water was sprayed on the blend consisting of atorvastatin calcium, microcrystalline cellulose, calcium carbonate, lactose monohydrate (granulated lactose) and half of the indicated amount of croscarmellose sodium in a fluid bed granulator. The generated granules were sieved and mixed with the remaining amount of croscarmellose sodium for 10 min.

Then the compression mass was lubricated with sieved magnesium stearate for another 5 min and the resulting blend was compressed into tablet cores with a standard rotary compression machine. All components for film coating were dissolved in water as solvent and then sprayed on the obtained tablet cores.

### Example 7:

| | **Function** | **Amount (mg)** |
|---|---|---|
| **Granules** | | |
| Atorvastatin Calcium | Active ingredient | 82.72* |
| Microcrystalline cellulose (Avicel PH 102)** | Filler | 52.28 |
| Calcium carbonate | Additive | 260.00 |
| Lactose monohydrate (Tablettose 70)*** | Filler | 60.00 |
| Croscarmellose Sodium (Ac-Di-Sol) | Disintegrant | 15.00 |
| Hydroxypropylcellulose (Klucel EF) | Binder | 10.00 |
| Tween 80 | Surfactant | 2.00 |
| Water | Solvent | q.s. |
| **External phase** | | **482.00** |
| Croscarmellose Sodium (Ac-Di-Sol) | Disintegrant | 15.00 |
| Magnesium Stearate | Lubricant | 3.00 |
| | | **500.00** |
| **Film/Coating** | | |
| Hydroxypropylmethylcellulose (Pharmacoat 606) | Coating polymer | 7.00 |
| Polyethylene glycol (PEG 6000 Powder) | Plasticizer | 2.00 |
| Titanium Dioxide | Coloring agent | 1.00 |
| Water | Solvent | q.s. |
| | | **10.00** |
| **Sum** | | **510.00** |

| | | |
|---|---|---|
| * corresponding to 80 mg atorvastatin substance without calculating calcium ** mean particle size of 100 µm *** ≤ 6% of particles have a particle size of <63 µm; 30-70% of particles have a particle size of <200 µm; ≥ 98% of particles have a particle size of <500 µm | | |

The cores of the Example 7 had the friability of 0.13 % and an abrasion of 0.18 %.

The tablets of Example 7 exhibit the mass variation of 103.8 %, content uniformity of 102.4 %, and a disintegration time of less than 5 min. The average weight of 20 tablets was 509.2 mg. Uniformity of mass conformed with the acceptance criteria defined in Ph. Eur.2.9.40. The diameter of the tablet was 11.6 mm, and the height of the tablet was 4.5 mm.

### Example 8:

| | **Function** | **Amount (mg)** |
|---|---|---|
| **Core** | | |
| Atorvastatin Calcium | Active ingredient | 82.72* |
| Microcrystalline cellulose (Avicel PH 102)** | Filler | 122.28 |
| Calcium carbonate | Additive | 130.00 |
| Lactose monohydrate (Tablettose 70)*** | Filler | 120.00 |
| Croscarmellose Sodium (Ac-Di-Sol) | Disintegrant | 30.00 |
| Hydroxypropylcellulose (Klucel EF) | Binder | 10.00 |
| Tween 80 | Surfactant | 2.00 |
| Magnesium Stearate | Lubricant | 3.00 |
| | | **500.00** |
| **Film/Coating** | | |
| Hydroxypropylmethylcellulose (Pharmacoat 606) | Coating polymer | 7.00 |
| Polyethylene glycol (PEG 6000 Powder) | Plasticizer | 2.00 |
| Titanium Dioxide | Coloring agent | 1.00 |
| Water | Solvent | q.s. |
| | | **10.00** |
| **Sum** | | **510.00** |

| | | |
|---|---|---|
| * corresponding to 80 mg atorvastatin substance without calculating calcium ** mean particle size of 100 µm *** ≤ 6% of particles have a particle size of <63 µm; 30-70% of particles have a particle size of <200 µm; ≥ 98% of particles have a particle size of <500 µm | | |

The cores of the Example 8 had the friability of 0.08 % and an abrasion of 0.20 %. The PSD d(50) of the compression mass was 175 µm.

The tablets of Example 8 exhibit the mass variation of 98.5 %, content uniformity of 98.9 %, and a disintegration time of less than 5 min. The average weight of 20 tablets was 501.5 mg. Uniformity of mass conformed with the acceptance criteria defined in Ph. Eur.2.9.40. The diameter of the tablet was 11.6 mm, and the height of the tablet was 4.8 mm.

### Example 9:

| | **Function** | **Amount (mg)** |
|---|---|---|
| **Core** | | |
| Atorvastatin Calcium | Active ingredient | 82.72* |
| Microcrystalline cellulose (Avicel PH 102)** | Filler | 122.28 |
| Calcium carbonate (Magnesia 4491, heavy) | Additive | 130.00 |
| Lactose monohydrate (Tablettose 70)*** | Filler | 120.00 |
| Croscarmellose Sodium (Ac-Di-Sol) | Disintegrant | 30.00 |
| Hydroxypropylcellulose (Klucel EF) | Binder | 10.00 |
| Tween 80 | Surfactant | 2.00 |
| Magnesium Stearate | Lubricant | 3.00 |
| | | **500.00** |
| **Film/Coating** | | |
| Hydroxypropylmethylcellulose (Pharmacoat 606) | Coating polymer | 7.00 |
| Polyethylene glycol (PEG 6000 Powder) | Plasticizer | 2.00 |
| Titanium Dioxide | Coloring agent | 1.00 |
| Water | Solvent | q.s. |
| | | **10.00** |
| **Sum** | | **510.00** |

| | | |
|---|---|---|
| * corresponding to 80 mg atorvastatin substance without calculating calcium ** mean particle size of 100 µm *** ≤ 6% of particles have a particle size of <63 µm; 30-70% of particles have a particle size of <200 µm; ≥ 98% of particles have a particle size of <500 µm | | |

The cores of the Example 9 had the friability of 0.03 % and an abrasion of 0.18 %. The PSD d(50) of the compression mass was 94 µm.

The average weight of 20 tablets of Example 9 was 507.5 mg. Uniformity of mass conformed with the acceptance criteria defined in Ph. Eur.2.9.40.

### Example 10:

Example 10 was prepared in the same manner as Example 1. Avicel PH 101 was used instead of Avicel PH 102.

| | **Function** | **Amount (mg)** |
|---|---|---|
| **Core** | | |
| Atorvastatin Calcium | Active ingredient | 82.72* |
| Microcrystalline cellulose (Avicel PH 101)** | Filler | 307.28 |
| Calcium carbonate | Additive | 50.00 |
| Fumed silica (Aerosil 200) | Glidant | 7.60 |
| Polyvinylpyrrolidone (Kollidon 25) | Binder | 6.80 |
| Sodium Starch Glycolate (Primojel) | Disintegrant | 33.60 |
| Magnesium Stearate | Lubricant | 12.00 |
| | | **500.00** |
| **Film/Coating** | | |
| Hydroxypropylmethylcellulose (Pharmacoat 606) | Coating polymer | 3.40 |
| Polyethylene glycol (PEG 6000 Powder) | Plasticizer | 1.00 |
| Titanium Dioxide | Coloring agent | 0.50 |
| Tween 80 | Surfactant | 5.10 |
| Water | Solvent | q.s. |
| | | **10.00** |
| **Sum** | | **510.00** |

| | | |
|---|---|---|
| * corresponding to 80 mg atorvastatin substance without calculating calcium ** mean particle size of 50 µm | | |

The PSD d(50) of the compression mass was 51 µm.

### Example 11:

Example 11 was prepared in the same manner as Example 7. Avicel PH 101 was used instead of Avicel PH 102 and Granulac 230 was used instead of Tablettose 70.

| | **Function** | **Amount (mg)** |
|---|---|---|
| **Granules** | | |
| Atorvastatin Calcium | Active ingredient | 82.72* |
| Microcrystalline cellulose (Avicel PH 101)** | Filler | 122.28 |
| Calcium carbonate | Additive | 130.00 |
| Lactose monohydrate (Granulac 230)*** | Filler | 120.00 |
| Hydroxypropylcellulose (Klucel EF) | Binder | 10.00 |
| Tween 80 | Surfactant | 2.00 |
| Water | | |
| **External phase** | | |
| Croscarmellose Sodium (Ac-Di-Sol) | Disintegrant | 30.00 |
| Magnesium Stearate | Lubricant | 3.00 |
| | | **500.00** |
| **Film/Coating** | | |
| Hydroxypropylmethylcellulose (Pharmacoat 606) | Coating polymer | 7.00 |
| Polyethylene glycol (PEG 6000 Powder) | Plasticizer | 2.00 |
| Titanium Dioxide | Coloring agent | 1.00 |
| Water | Solvent | q.s. |
| | | **10.00** |
| **Sum** | | **510.00** |

| | | |
|---|---|---|
| * corresponding to 80 mg atorvastatin substance without calculating calcium ** mean particle size of 50 µm ***≥90% of particles have a particle size of <63 µm | | |

The PSD d(50) of the compression mass was 66.2 µm.

The compositions of lower strengths comprising 10, 20 and 40 mg of atorvastatin were proportional to the 80 mg strength, respectively using proportional lower amounts of the excipients accordingly. The core weight and the weight of the coated tablets were as follows:

| | **10 mg** | **20 mg** | **40 mg** | **80 mg** |
|---|---|---|---|---|
| Core weight [mg] | 62.5 | 125.0 | 250.0 | 500.0 |
| Weight of film coated tablet [mg] | 65.0 | 128.5 | 256.0 | 510.0 |

### Comparative Examples

Comparative examples 1 and 2 were prepared using fluid bed granulation method.

A granulation solution consisting of hydroxypropylcellulose and Tween 80 dissolved in water was sprayed on the blend consisting of atorvastatin calcium, microcrystalline cellulose, calcium carbonate, lactose monohydrate and half of the indicated amount of croscarmellose sodium in a fluid bed granulator. The generated granules were sieved and mixed with the remaining amount of croscarmellose sodium for 10 min.

Then the compression mass was lubricated with sieved magnesium stearate for another 5 min and the resulting blend was compressed into tablet cores with a standard rotary compression machine. All components for film coating were dissolved in water as solvent and then sprayed on the obtained tablet cores.

### Comparative Example 1:

| | **Function** | **Amount (mg)** |
|---|---|---|
| **Granules** | | |
| Atorvastatin Calcium | Active ingredient | 82.72* |
| Microcrystalline cellulose (Emcocel 90)** | Filler | 168.08 |
| Calcium carbonate | Additive | 260.00 |
| Lactose monohydrate (Tablettose 70)*** | Filler | 218.00 |
| Croscarmellose Sodium (Ac-Di-Sol) | Disintegrant | 24.00 |
| Hydroxypropylcellulose (Klucel EF) | Binder | 16.00 |
| Tween 80 | Surfactant | 3.20 |
| **External phase** | | |
| Croscarmellose Sodium (Ac-Di-Sol) | Disintegrant | 24.00 |
| Magnesium Stearate | Lubricant | 4.00 |
| | | **800.00** |
| **Film/Coating** | | |
| Hydroxypropylmethylcellulose (Pharmacoat 606) | Coating polymer | 14.00 |
| Polyethylene glycol (PEG 6000 Powder) | Plasticizer | 4.00 |
| Titanium Dioxide | Coloring agent | 2.00 |
| Water | Solvent | q.s. |
| | | **20.00** |
| **Sum** | | **820.00** |

| | | |
|---|---|---|
| * corresponding to 80 mg of atorvastatin substance without calculating calcium ** mean particle size of 133 µm ***≤ 6% of particles have a particle size of <63 µm; 30-70% of particles have a particle size of <200 µm; ≥ 98% of particles have a particle size of <500 µm | | |

The cores of the Comparative Example 1 had the friability of 0.18 % and an abrasion of 0.37 %. The PSD d(50) of the compression mass was 142 µm.

The tablets of Comparative Example 1 exhibit the mass variation of 100.5 %, content uniformity of 98.8 %, and a disintegration time of less than 5 min. The average weight of 20 tablets was 806.0 mg. Uniformity of mass conformed with the acceptance criteria defined in Ph. Eur.7.0, Chapter 2.9.40. The diameter of the tablet was 13.2 mm, and the height of the tablet was 5.5 mm.

### Comparative Example 2:

A granulation solution consisting of hydroxypropylcellulose and Polysorbate 80 dissolved in water was sprayed on the blend consisting of atorvastatin calcium, microcrystalline cellulose, calcium carbonate, lactose monohydrate and croscarmellose sodium in a fluid bed granulator. The generated granules were sieved and then the compression mass was lubricated with sieved magnesium stearate for another 5 min and the resulting blend was compressed into tablet cores with a standard rotary compression machine. All components for film coating were dissolved in water as solvent and then sprayed on the obtained tablet cores.

| | **Function** | **Amount (mg)** |
|---|---|---|
| **Core** | | |
| Atorvastatin Calcium | Active ingredient | 82.72* |
| Microcrystalline cellulose (Emcocel 90)** | Filler | 176.08 |
| Calcium carbonate | Additive | 260.00 |
| Lactose monohydrate (Tablettose 70)*** | Filler | 218.00 |
| Croscarmellose Sodium (Ac-Di-Sol) | Disintegrant | 24.00 |
| Hydroxypropylcellulose (Klucel EF) | Binder | 32.00 |
| Tween 80 | Surfactant | 3.20 |
| Magnesium Stearate | Lubricant | 4.00 |
| | | **800.00** |
| **Film/Coating** | | |
| Hydroxypropylmethylcellulose (Pharmacoat 606) | Coating polymer | 14.00 |
| Polyethylene glycol (PEG 6000 Powder) | Plasticizer | 4.00 |
| Titanium Dioxide | Coloring agent | 2.00 |
| Water | Solvent | q.s. |
| | | **20.00** |
| **Sum** | | **820.00** |

| | | |
|---|---|---|
| * corresponding to 80 mg of atorvastatin substance without calculating calcium ** mean particle size of 133 µm ***≤ 6% of particles have a particle size of <63 µm; 30-70% of particles have a particle size of <200 µm; ≥ 98% of particles have a particle size of <500 µm | | |

The cores of the Comparative Example 2 had the friability of 0.00 % and an abrasion of 0.20 %. The PSD d(50) of the compression mass was 186 µm.

The tablets of Comparative Example 2 exhibit the mass variation of 97.5 %, content uniformity of 97.9 %, and a disintegration time of 12 min. The average weight of 20 tablets was 820.2 mg. Uniformity of mass conformed within the acceptance criteria defined in Ph. Eur.2.9.40. The diameter of the tablet was 13.2 mm, and height of the tablet was 5.5 mm.

### Comparative Example 3: Lipitor 80 mg commercial product

A commercial product of tablets comprising 80 mg of atorvastatin Ca was retrieved from the US and UK market. The tablets from the US market had the weight of 1241 mg, were oval and had a tablet width of 10.4 mm and a tablet length of 19.3 mm. Said tablets had disintegration time of less than 4 min.

The tablets of the commercial product of the UK market had the weight of 822 mg, were round and had diameter of 12.1 mm and had a disintegration time of less than 5 min.

### Methods were used for determining the parameters

Uniformity of mass variation, mass uniformity, content uniformity was determined and disintegration time were measured on the final tablets. The following methods we used:
- Mass variation was determined according to Pharm. Eur.7.0, Chapter 2.9.40.
- Uniformity of mass was determined according to Pharm. Eur.7.0, Chapter 2.9.5.
- Content uniformity was determined according to Pharm. Eur.7.0, Chapter 2.9.6.
- Disintegration time was determined according to Pharm. Eur.7.0, Chapter 2.9.1.

Friability and abrasion were measured on the cores of composition. Friability was determined according to Pharm. Eur.7.0, Chapter 2.9.7. Abrasion was determined in the same manner as friability, with the only difference of using a smaller drum having the outer diameter of 200 mm and having 12 projections.

Particle size distribution (PSD) was measured on the compression mass prior to compression of the compositions using the following measurement parameters: Sample weight: 300 mg; feeding rate: 40 %; Hopper Gap: 0.7 mm; Air pressure: 0.2 bar; Background measurement time: 12 sec; sample measurement time: 12 sec; Measurement device: Mastersizer 3000 with Aero S unit; Analysis model: Fraunhofer / General purpose /enhanced sensitivity

## Claims

1. Solid pharmaceutical composition comprising
from 10 wt % to 20 wt % atorvastatin or a pharmaceutically acceptable salt thereof,
from 5 wt % to 60 wt % of an additive selected from group consisting of alkali metal salts and alkaline earth metal salts, and
at least one further pharmaceutically acceptable excipient,
wherein the total weight of the composition is less than or equal to 600 mg.

2. The composition according to claim 1, wherein the weight amount of atorvastatin or a pharmaceutically acceptable salt thereof is about 80 mg and the total weight of the composition is from 300 mg to 600 mg, more preferably 450 to 550 mg, most preferably about 500 mg.

3. The composition according to claim 1, wherein the weight amount of atorvastatin or a pharmaceutically acceptable salt thereof is about 40 mg and the total weight of the composition is less than or equal to 300 mg, or the weight amount of atorvastatin or a pharmaceutically acceptable salt thereof is about 20 mg and the total weight of the composition is less than or equal to 150 mg or wherein the weight amount of atorvastatin or a pharmaceutically acceptable salt thereof is about 10 mg and the total weight of the composition is less than or equal to 75 mg.

4. The composition according to any of the preceding claims, wherein the pharmaceutically acceptable salt of atorvastatin is selected from the group consisting of salts with calcium, magnesium, potassium, aluminium, iron and zinc.

5. The composition according to any of the preceding claims, wherein the additive is selected from the group consisting of carbonates, bicarbonates, hydroxides, silicates, phosphates of sodium or calcium, and combinations thereof.

6. The composition according to claim 1 or 6, wherein the additive is selected from the group consisting of carbonates or bicarbonates of sodium or calcium, and combinations thereof.

7. The composition according to any of the preceding claims, wherein the at least one further pharmaceutically acceptable excipient is selected from the group consisting of a filler, a disintegrant, a binder, a glidant, a lubricant, a surfactant, and optionally further an antioxidant, a sweetener, a chelating agent, and combinations thereof.

8. The composition according to any of the preceding claims, wherein the at least one further pharmaceutically acceptable excipient is a filler having mean particle size of more than 50 µm.

9. The composition according to any of the preceding claims, wherein the composition having a particle size distribution d(50) equal or higher than 75 µm.

10. The composition according to any of the preceding claims, which is in the form of compressed tablet cores, in the form of pellets or in the form of granules.

11. Process for preparing the composition according to claim 10 comprising the steps of
a) providing atorvastatin or a pharmaceutically acceptable salt thereof, an additive selected from group consisting of alkali metal salts and alkaline earth metal salts, and at least one further pharmaceutically acceptable excipient;
b) mixing the components of step a);
c) preparing cores, pellets or granules comprising the composition.

12. The process according to claim 11, wherein the process for preparing the cores of step c) involves direct compression, pelletization or granulation.

13. Tablet comprising the composition according to claims 1 to 10 as a tablet core, further comprising a coating.

14. The tablet of claim 13 having a diameter of less than or equal to 12.0 mm.

15. The composition according to claims 1 to 10 or the tablet according to claims 13 and 14 for use in the treatment of hypercholesterolemia or hyperlipidemia.
